# EUROPÄISCHE PATENTSCHRIFT

(11) **EP 2 217 966 B1**
(45) Veröffentlichungstag und Bekanntmachung des Hinweises auf die Patenterteilung: **30.03.2016**
(21) Anmeldenummer: 08847471.3
(22) Anmeldetag: 07.11.2008
(51) Int. Cl.: G02F 1/39, G01N 33/22

(54) **NICHTLINEAR-OPTISCHER FREQUENZKONVERTER SOWIE VERWENDUNGEN DESSELBEN**
NON-LINEAR OPTICAL FREQUENCY CONVERTER AND USES THEREOF
CONVERTISSEUR DE FRÉQUENCE OPTIQUE NON LINÉAIRE, ET SES UTILISATIONS

(30) Priorität: 09.11.2007 DE 102007053542; 18.01.2008 DE 102008005129
(43) Veröffentlichungstag der Anmeldung: 18.08.2010
(73) Patentinhaber: Spherea GmbH, 89077 Ulm (DE)
(72) Erfinder: PEUSER, Peter, 85521 Riemerling (DE); FIX, Andreas, 82205 Gilching (DE)
(74) Vertreter: Mazabraud, Xavier
(86) Internationale Anmeldenummer: PCT/DE2008/001838
(87) Internationale Veröffentlichungsnummer: WO 2009/059598

(56) Entgegenhaltungen:
- WO-A-02/086946
- DE-A1- 4 219 169
- DE-A1- 19 634 161
- US-A- 5 117 126
- US-A- 5 742 626
- FIX A ET AL: "INTRACAVITY FREQUENCY MIXING IN PULSED OPTICAL PARAMETRIC OSCILLATORS FOR THE EFFICIENT GENERATION OF CONTINUOUSLY TUNABLE ULTRAVIOLET RADIATION" APPLIED PHYSICS B: LASERS AND OPTICS, SPRINGER INTERNATIONAL, BERLIN, DE, Bd. B67, Nr. 3, 1. September 1998 (1998-09-01), Seiten 331-338, XP000783011 ISSN: 0946-2171 in der Anmeldung erwähnt

## Beschreibung

Die Erfindung betrifft einen nichtlinear-optischen Frequenzkonverter mit einem optischen parametrischen Oszillator.

Gepulste in der Wellenlänge abstimmbare oder veränderliche Laserstrahlung wird in erster Linie durch Anwendung eines optischen nicht linearen Prozesses in einem sogenannten optischen parametrischen Oszillator - im Folgenden auch kurz OPO genannt - erzeugt.

Aus der US 5 117 126 A ist ein optischer parametrischer Oszillator bekannt, der zwei oder mehrere optisch nicht lineare Medien in einem einzelnen Resonator aufweist. Die beiden als Kristalle vorgesehenen Medien können unabhängig voneinander gedreht werden, um so eine abstimmbare Strahlung mit mehreren abstimmbaren Wellenlängen zu erzeugen. Zur Erzeugung einer schmalbandigen hochqualitativen Strahlung kann weiter ein zweiter OPO zum Liefern einer auf die OPO-Strahlung des ersten OPOs abgestimmten Injektionsstrahlung zum Durchführen eines Injection Seedings vorgesehen sein.

Abstimmbare OPO-Anordnungen sowie Verwendungen der selben sind beispielsweise aus der DE 199 23 005 B4, der DE 198 19 178 C2, der EP 1119796 B1 sowie der US 2006/0114464 A1 bekannt.

Die Erzeugung abstimmbarer Laserstrahlung hoher Strahlqualität mittels eines OPOs mit resonatorinterner Summenfrequenzerzeugung ist genauer beschrieben in A. Fix, G. Ehret; "Intracavity mixing in pulsed optical parametic oscillators for the efficient generation of continuously tunable ultraviolet radiation"; Appl. Phys. B 67 (1998) 331. Dieser Stand der Technik beschreibt einen Frequenzkonverter mit den Merkmalen des Oberbegriffs des beigefügten Anspruchs 1 sowie ein Verfahren zum Erzeugen abstimmbarer Laserstrahlung mit den Merkmalen des Oberbegriffs des beigefügten Anspruchs 13.

Es wird für weitere Einzelheiten zu dem Aufbau und der Verwendung solcher OPO-Anordnungen ausdrücklich auf die vorgenannten Druckschriften und Literaturstellen verwiesen.

Ein Nachteil der meisten bisher realisierten abstimmbaren OPO-Anordnungen ist dabei die geringe Strahlqualität sowie ein kleiner Wirkungsgrad, die im wesentlichen aus den physikalischen Eigenschaften der hierbei eingesetzten optisch nichtlinearen Kristalle resultieren.

Aufgabe der Erfindung ist es, einen nichtlinear-optischen Frequenzkonverter (oder auch Wellenlängenkonverter genannt) der im Oberbegriff des Anspruchs 1 genannten Art mit wesentlich breiterem Abstimmbereich zur Verfügung zu stellen. Diese Aufgabe wird durch einen nichtlinear-optischen Frequenzkonverter mit den Merkmalen des Patenanspruches 1 gelöst.

Vorteilhafte Ausgestaltungen der Erfindung sind Gegenstand der Unteransprüche. Ein insbesondere unter Einsatz eines solchen Frequenzkonverters durchführbares Verfahren zum Erzeugen von gepulster abstimmbarer Laserstrahlung ist im Anspruch 13 definiert. Weitere vorteilhafte Verwendungen des Frequenzkonverters sind Gegenstand der Nebenansprüche.

Der erfindungsgemäße nichtlinear-optische Frequenzkonverter weist zunächst einen ersten optisch parametrischen Oszillator auf. Dieser erste optisch parametrische Oszillator ist weiter erfindungsgemäß mit einer Frequenzmischereinrichtung mit einem Frequenzmischer-Kristall versehen. Weiter weist der erste optische parametrische Oszillator eine Einkoppeleinrichtung zum Einkoppeln von außerhalb des ersten optischen parametrischen Oszillators erzeugter Mischerstrahlung auf. Durch Einkoppeln der Mischerstrahlung mittels der Frequenzmischereinrichtung, die im Inneren des Resonators des ersten OPOs angeordnet ist, lässt sich mit hohem Wirkungsgrad auch eine hohe Strahlqualität für die hierbei entstehende Strahlung erreichen.

Weiter ist erfindungsgemäß eine abstimmbare Strahlquelle zur Lieferung abstimbarer Strahlung vorgesehen, welche als Mischerstrahlung über die Einkoppeleinrichtung in die Frequenzmischereinrichtung einkoppelbar ist. Durch das Einkoppeln und das Mischen der abstimmbaren Mischerstrahlung lässt sich zusätzlich zu dem Vorteil einer hohen Strahlqualität auch der Abstimmbereich der neu zu erzeugenden Wellenlängen oder Frequenzen wesentlich erweitern. Damit ist bei hoher Strahlqualität auch eine große Abstimmbreite gewährleistet.

Auch lässt sich mit einer solchen Konfiguration der ansonsten technisch höchst schwierige und problematische Bereich besonders niedriger Wellenlängen bei bzw. unterhalb von etwa 200 nm erschliessen.

In besonders bevorzugter Ausgestaltung der Erfindung ist der Frequenzkonverter ein gepulster nichtlinear-optischer Frequenzkonverter, dessen erster optisch parametrischer Oszillator mit einem resonatorinternen Frequenzmischer-Kristall als Frequenzmischereinrichtung versehen ist. Die Einkoppeleinrichtung weist vorzugsweise einen breitbandigen Einkoppelspiegel zum Einkoppeln der abstimmbaren Mischerstrahlung auf. Als weitere Strahlquelle zum Liefern der abstimmbaren Mischerstrahlung mit hoher Qualität wird erfindungsgemäß ein zweiter, weiter vorzugsweise zeitgleich gepumpter oder synchronisierter, abstimmbarer OPO eingesetzt. Dessen abstimmbare Strahlung lässt sich über die vorzugsweise als breitbandiger Einkoppelspiegel ausgebildete Einkoppeleinrichtung als Mischerstrahlung in die Frequenzmischereinrichtung einkoppeln.

Der zweite abstimmbare OPO ist vorzugsweise ein OPO mit resonatorinterner Summenfrequenzerzeugung, da hierbei eine hohe Strahlqualität bereitgestellt wird, die für einen effizienten Prozess der Frequenzmischung und für die Erzeugung einer hieraus resultierenden Strahlung mit hoher Strahlqualität vorteilhaft ist.

Bei Pulsbetrieb ist es wünschenswert, dass der Puls der beizumischenden Mischerstrahlung zeitgleich mit dem Puls der OPO-Strahlung des ersten OPOs ist. Bevorzugt ist hierzu die abstimmbare Strahlquelle synchron mit dem ersten OPO gekoppelt. Dies lässt sich vorzugsweise dadurch erreichen, dass die abstimmbare Strahlquelle und der die Mischerstrahlung aufnehmende erste OPO von ein und derselben Pumpquelle, beispielsweise von ein und demselben Pumplaser angeregt werden. Vorzugsweise wird hierzu die Strahlung der Pumpquelle in geeigneter Weise auf beide Teilkonfigurationen aufgeteilt.

Für eine genaue Synchronisation ist in einigen Ausführungsformen eine optische Verzögerungsstrecke vorgesehen.

Erfindungsgemäß weist die Frequenzmischereinrichtung einen Frequenzmischer-Kristall auf.

Gemäß einer weiteren bevorzugten Ausgestaltung der Erfindung weist die Einkoppeleinrichtung einen Einkoppelspiegel zum Einkoppeln der abstimmbaren Mischerstrahlung in einen die Frequenzmischereinrichtung enthaltenden Resonator des ersten optisch parametrischen Oszillators auf.

In bevorzugter Ausgestaltung der Erfindung wird mittels eines speziellen Spiegels ein von einer zweiten Strahlquelle erzeugter, in der Wellenlänge abstimmbarer Strahl als beizumischende Mischerstrahlung in einen Resonator des OPOs eingekoppelt. Dieser in dieser Ausgestaltung als Einkoppeleinrichtung verwendete Einkoppelspiegel ist vorzugsweise so beschaffen, dass einerseits die in dem Resonator umlaufende OPO-Strahlung, also z.B. eine Signalstrahlung oder Idler-Strahlung, nicht oder nur gerinfügig beeinträchtigt wird. Andererseits sollte der Einkoppelspiegel eine Wellenlängen-Bandbreite aufweisen, welche zumindest einem Großteil der Bandbreite der einzukoppelnden Mischerstrahlung entspricht. In bevorzugter Ausgestaltung entspricht die Wellenlängen-Bandbreite des Einkoppelspiegels mindestens der Bandbreite der einzukoppelnden Mischerstrahlung.

In einer ersten vorteilhaften Ausgestaltung der Erfindung hat der Einkoppelspiegel hierzu eine hohe Reflexion nahe 100 % (z.B. mehr als 90 % und insbesondere mehr als 95 %, beispielsweise mehr als 99 %) für den Wellenlängenbereich der OPO-Strahlung in dem ersten OPO und eine große Transmission für den Wellenlängenbereich der einzukoppelnden Mischerstrahlung. In einer zweiten vorteilhaften Ausgestaltung hat der Einkoppelspiegel eine Transmission nahe 100 % für den Wellenlängenbereich der OPO-Strahlung in dem ersten OPO und eine große Reflektion für den Wellenlängenbereich der einzukoppelnden Mischerstrahlung.

In beiden Fällen steht der Einkoppelspiegel vorzugsweise unter einem Winkel von etwa 45° zur OPO-Strahlung in dem ersten OPO.

Die zweite vorteilhafte Ausgestaltung ist besonders bevorzugt, wenn externe Mischerstrahlung im Wellenlängenbereich unterhalb von 300 nm eingekoppelt werden soll. Für einen auf solche Wellenlängen ausgelegten Spiegel geeignete optische Schichten lassen sich in haltbarer Weise weitaus besser mit hoher Reflexion als mit hoher Transmission herstellen.

Für den OPO und den Frequenzkonverter kommen insbesondere alle Kristalle in Frage, die aufgrund ihrer physikalischen Eigenschaften für solche Zwecke ausgebildet sind. Zum Beispiel können als optisch-nichtlineare Kristalle für den optischparametrischen Ostillator sowie für die Freqenzmischung alle nach dem Stand der Wissenschaft hierfür bekannten Kristalle vorgesehen sein, insbesondere KTP, KTA, BBO, LBO, LiNb0₃. Nähere Ausführungen hierzu finden sich in V.G. Dimitriev et al., Handbook of Nonlinear Optical Crystals, Springer-Verlag 1991. Es wird für nähere Einzelheiten ausdrücklich auf diese Literaturstelle verwiesen.

Vorteilhafte Anwendungen oder Verwendungen der Erfindung sind eine Laseranordnung zur Erzeugung gepulster abstimmbarere Laserstrahlung. Insbesondere lässt sich damit ein UV-Lasersystem aufbauen, das für eine selektive und effiziente Ionisierung von Molekülen geeignet ist. Eine solche Ionisierung ist insbesondere für den empfindlichen Nachweis von Explosivstoffen oder anderen Gefahrstoffen mittels lonenmobilitätsspektrometrie verwendbar. Demgemäß ist als ein Einsatzgebiet des Frequenzkonverters ein lonenmobilitätsspektrometrieverfahren vorgesehen, bei dem eine Laseranordnung mit einem erfindungsgemäßen Frequenzkonverter verwendet wird.

Andererseits lässt sich der erfindungsgemäße Konverter auch in LIDAR-Anordnungen einsetzen. Z.B. ist der Frequenzkonverter in einem Transmitter für LIDAR-Systeme einsetzbar.

Ausführungsbeispiele der Erfindung werden nachfolgend anhand der beigefügten Zeichnungen näher erläutert. Darin zeigt:
- Fig. 1: eine schematische Ansicht eines gepulsten nichtlinear-optischen Frequenzkonverters mit einem resonatorinternen Frequenzmischer-Kristall, in den eine externe Mischerstrahlung eingekoppelt wird, wobei eine erste Ausführungsform einer Einkoppeleinrichtung eingesetzt ist;
- Fig. 2: eine schematische Ansicht eines Frequenzkonverters vergleichbar derjenigen von Fig. 1, bei dem eine zweite Ausführungsform einer Einkoppeleinrichtung verwendet wird;
- Fig. 3: eine Ansicht vergleichbar derjenigen von Fig. 2, wobei ein separater Spiegel zur Auskoppelung einer Summenfrequenzstrahlung verwendet ist;
- Fig. 4: eine schematische Darstellung einer ersten Ausführungsform eines nichtlinear-optischen Frequenzkonverters mit breitem Abstimmbereich und hoher Strahlqualität, wobei als Strahlungsquelle zum Liefern der einzukoppelnden Mischerstrahlung eine abstimmbare Strahlungsquelle verwendet wird und weiter eine breitbandige Einkoppeleinrichtung in einer zu der ersten Ausführungsform von Fig. 1 vergleichbaren Konfiguration eingesetzt ist;
- Fig. 5: eine schematische Darstellung einer zweiten Ausführungsform des nicht linear-optischen Frequenzkonverters, mit einer breitbandigen Einkoppeleinrichtung in einer zu der zweiten Ausführungsform von Fig. 2 vergleichbaren Konfiguration;
- Fig. 6: eine schematische Darstellung einer dritten Ausführungsform des nichtlinear-optischen Frequenzkonverters mit einer separaten Auskoppeleinrichtung vergleichbar zu derjenigen von Fig. 3;
- Fig. 7: ein Diagramm zur Darstellung von resultierenden Wellenlängen, die mit Frequenzkonverter-Konfigurationen gemäß einer der Ausgestaltungen der Fig. 4 - 6 erzeugbar sind; und
- Fig. 8: ein weiteres Diagramm zur Darstellung von resultierenden Wellenlängen, die mit einer Frequenzkonverter-Konfiguration gemäß einer der Abbildungen 4 - 6 erzeugbar sind.

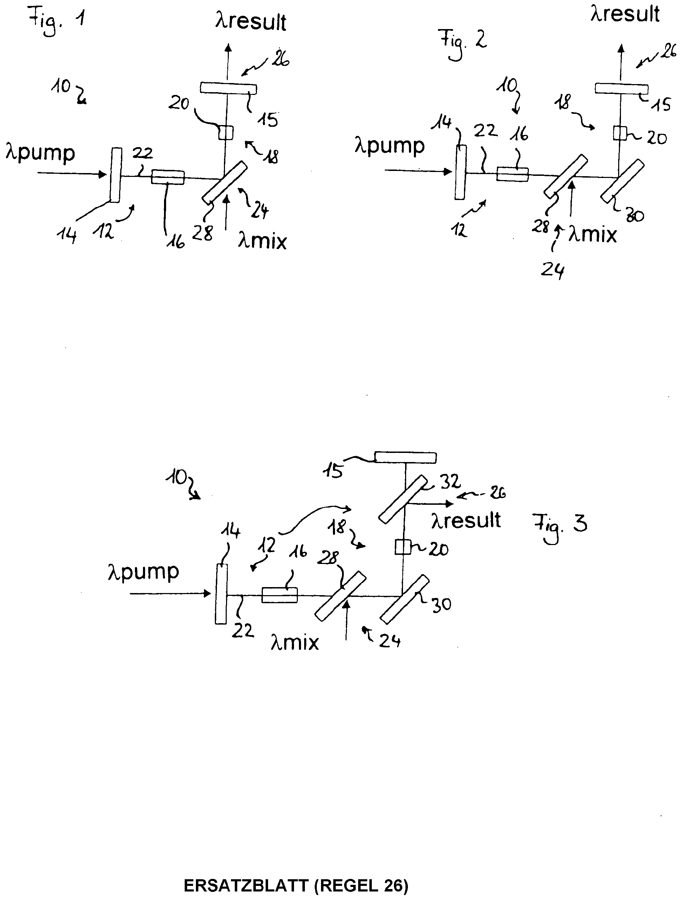

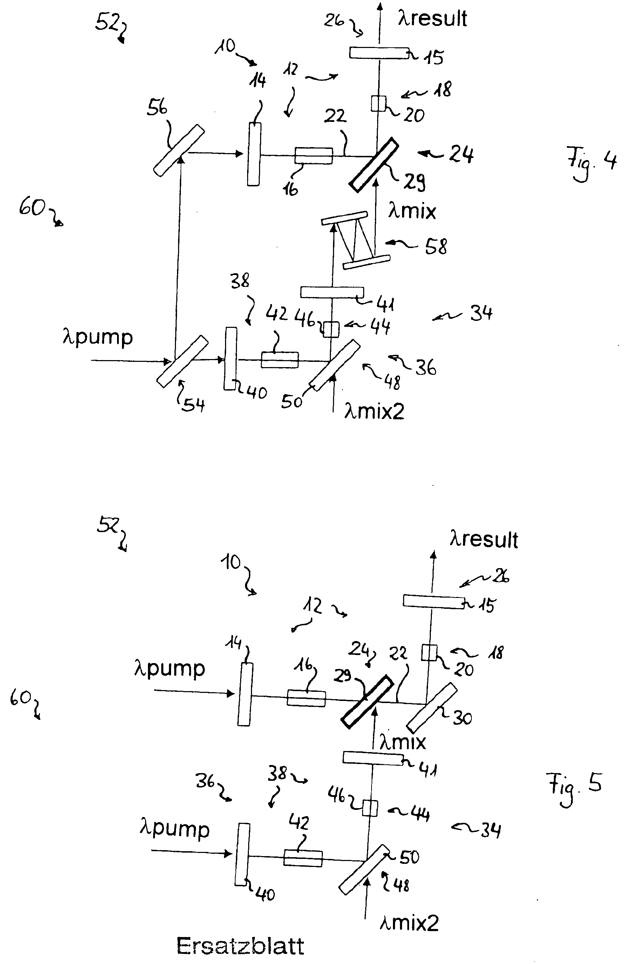

Im Folgenden wird zunächst anhand der Fig. 1 - 3 an drei beispielhaften Ausführungsformen das Prinzip einer Summenfrequenzerzeugung mit einer extern erzeugten Strahlung - Mischerstrahlung - in einem optisch parametrischen Oszillator erläutert, bevor anhand der Fig. 4 - 6 drei Ausführungsformen eines nichtlinear-optischen Frequenzkonverters erläutert werden, bei dem abstimmbare Mischerstrahlung zur Summenfrequenzerzeugung in den OPO eingekoppelt wird.

Die Fig. 1 - 3 zeigen unterschiedliche Ausführungsformen eines ersten optisch parametrischen Oszillators 10, mit dem gepulste abstimmbare Laserstrahlung λᵣₑₛᵤₗₜ durch Anwendung eines optisch nichtlinearen Prozesses erzeugbar ist.

Ein optisch parametrischer Oszillator (OPO) ist ein parametrischer Oszillator, der bei optischen Frequenzen oszilliert. Er konvertiert eine Pumpstrahlung λₚᵤₘₚ, meist eine Eingangslaserstrahlung, in wenigstens zwei Ausgangsstrahlungen niedrigerer Frequenz mittels nichtlinearer optischer Wechselwirkung. Aus historischen Gründen werden die beiden Ausgangsstrahlungen "Signalstrahlung" und "Idlerstrahlung" genannt. Ein OPO, wie hier der erste optische parametrische Oszillator 10, weist einen optischen Resonator 12, hier mit zwei Resonatorspiegeln 14, 15 und einen nichtlinearen optischen Kristall - OPO-Kristall 16 - auf. Der Resonator 12 ist dazu ausgebildet, wenigstens eine der beiden Strahlungen - Signalstrahlung oder Idlerstrahlung - zwischen den Resonatorspiegeln 14, 15 hin und her schwingen zu lassen.

Im Inneren des Resonators 12, zwischen den Resonatorspiegeln 14, 15, ist eine Frequenzmischereinrichtung 18 mit einem Mischer-Kristall 20 vorgesehen. Der Mischer-Kristall 20 ist ein nichtlinearer Kristall, in dem eine Summenfrequenzerzeugung der in dem Resonator 12 schwingenden OPO-Strahlung 22 mit einer extern erzeugten Strahlung - Mischerstrahlung λₘᵢₓ - erfolgt. Dadurch kann mit einem hohen Wirkungsgrad auch eine Strahlqualität für die hierbei entstehende Strahlung λᵣₑₛᵤₗₜ erreicht werden. Dabei wird eine der beiden OPO-Strahlungen 22-- beispielsweise die Signalstrahlung λ_{signal} - mit einem von außen zugeführten Strahl der Mischerstrahlung λₘᵢₓ in dem nichtlinearen Mischer-Kristall 20 gemischt.

Eine hohe Strahlqualität lässt sich durch Einkoppeln der Mischerstrahlung λₘᵢₓ mit hoher Strahlqualität erreichen.

Zum Einkoppeln der Mischerstrahlung λₘᵢₓ wird eine Einkoppeleinrichtung 24 verwendet. Zum Auskoppeln der resultierenden Strahlung λᵣₑₛᵤₗₜ wird eine Auskoppeleinrichtung 26 verwendet. Die Ausführungsformen der Fig. 1 - 3 unterscheiden sich durch unterschiedliche Ausgestaltungen der Einkoppeleinrichtung 24 und der Auskoppeleinrichtung 26.

Zum Einkoppeln weist die Einkoppeleinrichtung 24 einen Einkoppelspiegel 28 auf. Bei der Ausgestaltung der Fig. 1 hat der Einkoppelspiegel 28 eine hohe Reflexion nahe 100 % für den Wellenlängenbereich der OPO-Strahlung 22 und eine große Transmission für die Mischerstrahlung λ_{mix.} Der Einkoppelspiegel 28 steht vorzugsweise unter einem Winkel von 45° zur OPO-Strahlung 22. Bei der Ausgestaltung gemäß den Figuren 2 und 3 hat der Einkoppelspiegel 28 eine hohe Transmission nahe 100 % für den Wellenlängenbereich der OPO-Strahlung 22 und eine große Reflexion für die Mischerstrahlung λ_{mix.} Der Spiegel steht auch hier unter einem Winkel von 45° zur OPO-Strahlung 22, wobei jedoch noch ein Umlenkspiegel 30 mit hoher Reflexion für beide Strahlungen 22, λₘᵢₓ vorgesehen ist. Während bei den Ausgestaltungen der Fig. 1 und 2 die Auskoppeleinrichtung 26 im wesentlichen durch einen teiltransparenten zweiten Resonatorspiegel 15 gebildet ist, ist bei der Ausgestaltung gemäß Fig. 3 die Auskoppeleinrichtung 26 noch mit einem zusätzlichen Auskoppelspiegel 32 versehen.

Wenn für die Mischerstrahlung λₘᵢₓ eine Strahlung fester Wellenlänge verwendet wird, können die Einkoppeleinrichtung 24 und insbesondere der Einkoppelspiegel 28 relativ einfach ausgebildet werden; der Nachteil einer solchen Konfiguration besteht allerdings darin, dass der Abstimmbereich der neu zu erzeugenden Wellenlängen der resultiernden Strahlung λᵣₑₛᵤₗₜ durch den Mischprozess reduziert ist und somit die Anwendung des ersten optisch parametrischen Oszillators 10 stark eingeschränkt ist. Die Gründe hierfür liegen einerseits in der technischen Herstellbarkeit von geeigneten OPO-Resonatorspiegeln 14, 15 mit größerer Bandbreite. Andererseits begrenzen die physikalischen Eigenschaften der OPO-Kristalle 16 den Abstimmbereich, wobei insbesondere eine große Absorption im langweiligen Bereich der Idler-Wellenlänge maßgeblich ist.

Verwendet man dagegen für die Mischerstrahlung λₘᵢₓ keine Strahlung fester Wellenlänge, sondern eine in der Wellenlänge (z.B. kontinuierlich) veränderbare oder abstimmbare Strahlung, dann lassen sich die vorgenannten Nachteile vermeiden, da die aufgeführten Einschränkungen des OPO-Betriebes hierbei nicht berührt werden. D.h., dass die OPO-Resonator-Spiegel 14, 15 sowie der OPO-Kristall 16 nicht verändert werden müssen und dennoch ein Wellenlängenkonverter oder ein Frequenzkonverter 52 entsteht, der bei hoher Strahlqualität auch eine große Abstimmbreite gewährleistet.

Darüber hinaus lässt sich aus den gleichen Gründen mit einer solchen Konfiguration der technisch schwierige Bereich besonders niedriger Wellenlängen bei bzw. unterhalb von 200 nm erschließen, wie im Folgenden noch näher dargestellt werden wird.

In einer ersten Ausgestaltung der Erfindung werden als Strahlquelle für die Mischerstrahlung λₘᵢₓ in Konfigurationen wie bei Fig. 1 - 3 jeweils ein abstimmbarer Laser (nicht näher dargestellt) verwendet, wobei anstelle der Einkoppelspiegel 28 entsprechend breitbandige Einkoppelspiegel 29 verwendet werden, die diese abstimmbare Mischerstrahlung λₘᵢₓ in ihrer gesamten Bandbreite durchlassen (wie in Fig. 1) bzw. reflektieren (wie in Fig. 2 und 3).

Besonders bevorzugte Ausführungsformen des Frequenzkonverters 52 werden im Folgenden anhand der Fig. 4 - 6 näher dargestellt, wobei für jeweils gleiche oder entsprechende Elemente die gleichen Bezugszeichen wie bei den Fig. 1 - 3 verwendet werden.

Bei den Ausführungsformen gemäß den Fig. 4 - 6 ist neben dem ersten optisch parametrischen Oszillator 10, der die Frequenzmischereinrichtung 18 mit dem Mischerkristall 20 enthält, und der Einkoppeleinrichtung 24 einschließlich einem Breitband-Einkoppelspiegel 29 noch eine abstimmbare Strahlquelle 34 zum Liefern einer in der Wellenlänge vorzugsweise kontinuierlich veränderbaren oder abstimmbaren Mischerstrahlung λₘᵢₓ vorgesehen.

Als abstimmbare Strahlquelle 34 ist bei den dargestellten Ausführungsformen gemäß den Fig. 4 - 6 ein zweiter optisch parametrischer Oszillator 36 vorgesehen.

Der zweite optisch parametrische Oszillator 36 ist ähnlich wie der erste optisch parametrische Oszillator 10 mit einem optischen Resonator 38, der zwei Resonatorspiegel 40, 41 aufweist, einem OPO-Kristall 42, einer Frequenzmischereinrichtung 44 mit Mischerkristall 46 und einer Einkoppeleinrichtung 48 mit einem Einkoppelspiegel 50 zum Einkoppeln einer Misch-Strahlung λₘᵢₓ₂ versehen.

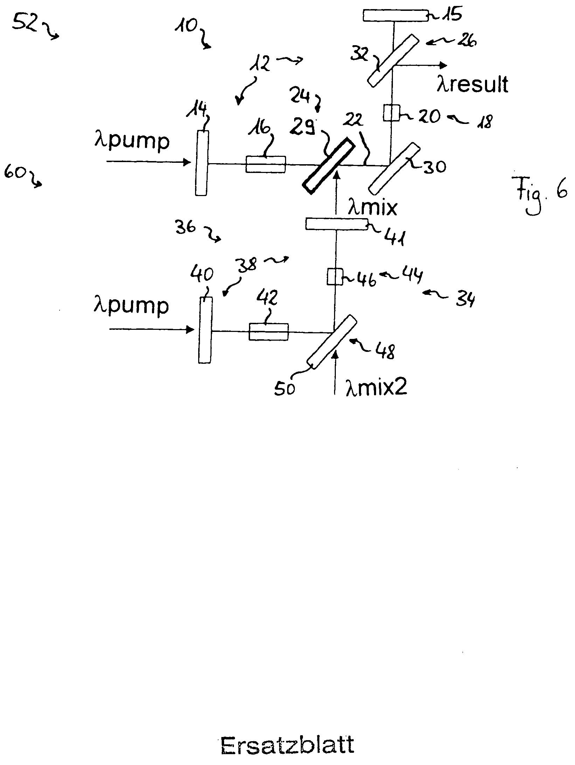

Die Ausführungsformen der Fig. 4, 5 und 6 unterscheiden sich entsprechend wie die Ausgestaltungen der Fig. 1 - 3 hinsichtlich ihrer Ausbildung und Anordnung der Einkoppeleinrichtung 24 sowie der Auskoppeleinrichtung 26 des ersten optisch parametrischen Oszillators 10. Die Fig. 4 entspricht hierbei der Ausgestaltung gemäß der Fig. 1, die Fig. 5 derjenigen von Fig. 2 und die Fig. 6 derjenigen von Fig. 3.

Die Strahlquelle 34 für die einzukoppelnde Mischerstrahlung λₘᵢₓ ist demnach vorzugsweise ein abstimmbarer OPO - zweiter optisch parametrischer Oszillator 36 - mit resonatorinterener Summenfrequenzerzeugung, da hierbei eine hohe Strahlqualität bereit gestellt wird, die für einen effizienten Prozess der Frequenzmischung und die Erzeugung einer hieraus resultierenden Strahlung mit hoher Strahlqualität vorteilhaft ist. Die Fig. 4 - 5 zeigen dabei Ausführungsbeispiele für entsprechende grundlegende Konfigurationen für einen breit abstimmbaren Frequenzkonverter 52.

Dabei sollte der Puls der beizumischenden Strahlung - Mischerstrahlung λₘᵢₓ - zeitgleich mit dem Puls der OPO-Strahlung 22 des ersten optisch parametrischen Oszillators 10 sein. Hierfür ist die externe Strahlquelle 34 mit dem ersten optisch parametrischen Oszillator 10 synchron gekoppelt.

Ein Ausführungsbeispiel hierzu ist in Fig. 4 dargestellt. Gemäß diesem Ausführungsbeispiel ist die externe Strahlquelle 34 und der die Mischerstrahlung λₘᵢₓ aufnehmende erste optisch parametrische Oszilator 10 von der Fundamentalen oder einer Harmonischen desselben Pumplaser (nicht explizit dargestellt; angedeutet durch die Pumpstrahlung λₚᵤₘₚ) angeregt, dessen Pumpstrahlung λₚᵤₘₚ in geeigneter Weise auf beide Teilkonfigurationen aufgeteilt wird. Bei dem Ausführungsbeispiel gemäß Fig. 4 ist hierzu ein Strahlteiler 54 (z.B semitransparenter Spiegel) und eine Umlenkeinrichtung 56 (z.B. Umlenkspiegel) vorgesehen.

Je nach Lichtverlauf kann es für eine genaue Synchronisation erforderlich sein, eine optische Verzögerungsstrecke 58 bei einer der beiden Teilkonfigurationen vorzusehen. In dem Ausführungsbeispiel ist die optische Verzögerungstrecke 58 zwischen die externe Strahlungsquelle 34 und den die Mischerstrahlung λₘᵢₓ aufnehmenden ersten OPO 10 eingebaut.

Wie oben bereits bei den Ausführungen gemäß der Fig. 1 - 3 erläutert, kann aber auch anstelle eines zweiten optisch parametrischen Oszillators 36 jede andere abstimmbare Strahlquelle für die Lieferung der Mischerstrahlung λₘᵢₓ eingesetzt werden, beispielsweise ein abstimmbarer Laser. Hierzu sollten möglichst die Synchronisation und eine hohe Strahlqualität gewährleistet werden.

Durch die beschriebenen Maßnahmen wird erreicht, dass
a) der Abstimmbereich des Frequenzkonverters 52 mit resonatorinterner Summenfrequenzerzeugung erheblich vergrößert wird und
b) die technisch besonders schwierige Erzeugung sehr kurzer Wellenlängen unterhalb von 200 nm wesentlich erleichtert wird.

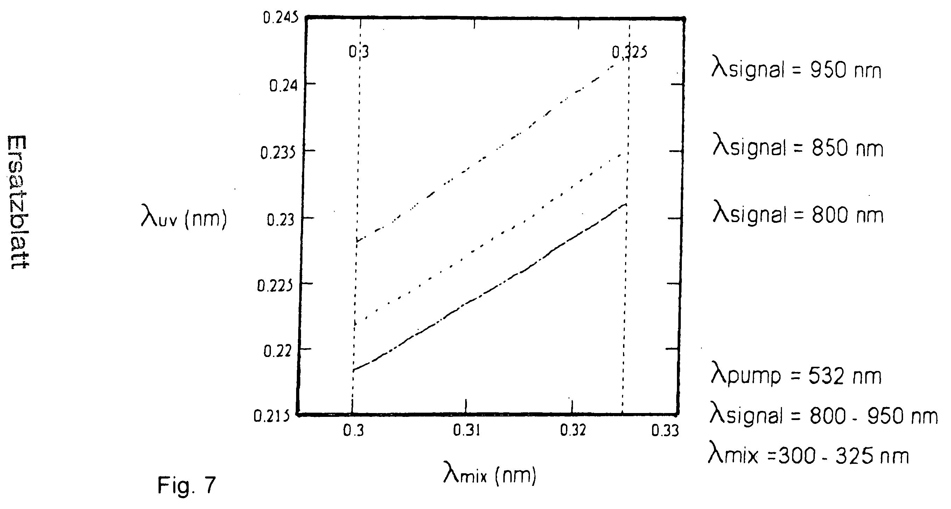

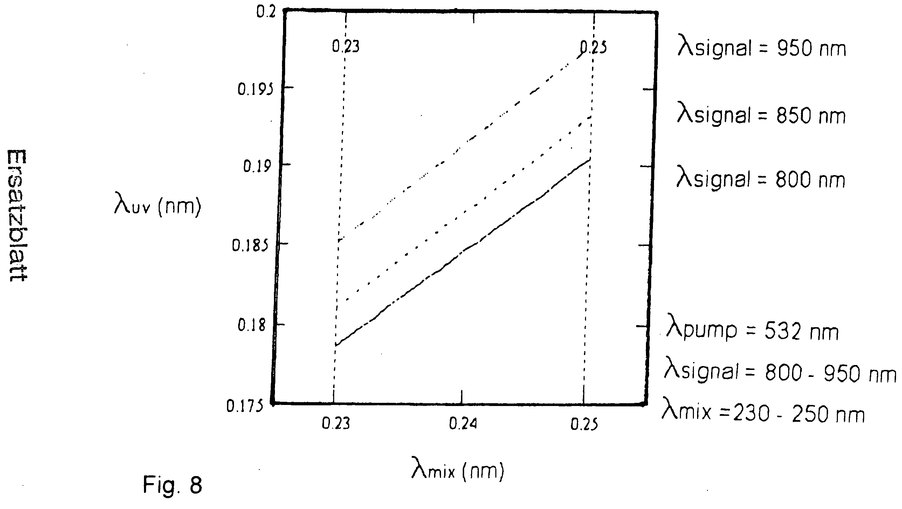

Dies wird anhand der Fig. 7 und 8 erläutert, mit denen Rechenergebnisse für die Ausgangsstrahlung λᵣₑₛᵤₗₜ, hier dargestellt als λᵤᵥ für eine Wellenlänge im UV-Bereich, der hier erläuterten Wellenlängenkonverter oder Frequenzkonverter 52 mit resonatorinterner Summenfrequenzerzeugung dargestellt werden.

Für die Mischerstrahlung λₘᵢₓ wurde ein Wellenlängenbereich zugrunde gelegt, welcher der mit Konfigurationen gemäß den Fig. 1 - 3 bei fester Wellenlänge λₘᵢₓ₂ der dort eingekoppelten Mischerstrahlung λₘᵢₓ₂ erzeugbaren Strahlung entspricht. In den Rechenbeispielen wurde für die Pumpstrahlung λₚᵤₘₚ die frequenzverdoppelte Strahlung von 532 nm des Hauptübergangs des Nd:YAG-Lasers verwendet. Die Signalstrahlung λ_{signal} reicht in diesem Falle von etwa 800 nm bis 950 nm.

Wie aus den Diagrammen ersichtlich, sind bei einer in den ersten OPO 10 einzukoppelnden Mischerstrahlung λₘᵢₓ zwischen 300 und 325 nm je nach Signalstrahlung λ_{signal} resultiernde UV-Strahlungen λᵤᵥ zwischen 215 und 245 nm erzielbar (s. Fig. 7). Koppelt man aber Mischerstrahlung λₘᵢₓ im Bereich von 230 - 250 nm ein, sind resultierende UV-Strahlungen λᵤᵥ im Bereich zwischen 175 und 200 nm erzielbar.

Anhand dieser Beispiele wird offenbar, dass der Abstimmbereich mit der neuen OPO-Konfiguration erheblich vergrößert wird.

Weiterhin ist aus den Fig. 7 und 8 zu erkennen, dass im Falle der Erzeugung von UV-Strahlung λᵤᵥ mit der hier vorgestellten Anordnung insbesondere auch der Bereich sehr kurzer Wellenlängen nahe bzw. unterhalb von 200 nm erschlossen werden kann, ohne dass die OPO-Resonatorspiegel 14, 15 bzw. 40, 41 oder der OPO-Kristall 16, 42 verändert werden müssen.

Der in den Fig. 4 - 6 gezeigte Frequenzkonverter 52 lässt sich in einer Laseranordnung 60 zur Erzeugung gepulster abstimmbarer Laserstrahlung λᵤᵥ verwenden.

Eine vorteilhafte Verwendung in einer solchen Laseranordnung 60 ist die Detektion von Gefahrstoffen mittels Ionenmobilitätsspektrometrie, beispielsweise in einem lonenmobilitätsspektrometer (nicht näher dargestellt). Ein lonenmobilitätsspektrometer (IMS) ist ein Spektrometer, das zur Detektion und Identifizierung von sehr geringen Konzentrationen von Chemikalien aufgrund der unterschiedlichen Migration von Ionen in einer Gasphase durch ein homogenes elektrisches Feld fähig ist. IMS-Vorrichtungen kommen in sehr unterschiedlichen Größen vor und sind oft für eine spezielle Anwendung angepasst. IMS-Vorrichtungen sind dazu fähig, unter unterschiedlichsten Umgebungsbedingungen zu arbeiten. Beispiele für lonenmobilitätsspektrometer finden sich in der WO 02/086946, auf die für weitere Einzelheiten ausdrücklich verwiesen wird. Die Laseranordnung 60 mit dem Frequenzkonverter 52 kann als UV-Lasersystem für die selektive und effiziente Ionisierung von Molekülen für den empfindlichen Nachweis von Explosivstoffen und anderen Gefahrstoffen mittels solcher lonenmobilitätsspektrometer eingesetzt werden.

Eine andere mögliche Anwendung sind LIDAR-Systeme, wo der Frequenzkonverter beispielsweise als LIDAR-Transmitter einsetzbar ist.

Beispiele für LIDAR-Systeme finden sich in der WO 2007/123 555, der US 2007/247612, der US 2007/210254 oder der US 2007/171397, auf die für weitere Einzelheiten zu LIDAR-Systemen ausdrücklich verwiesen wird.

### Bezugszeichenliste:

- 10: erster optisch parametrischer Oszillator
- 12: optischer Resonator
- 14: erster Resonatorspiegel
- 15: zweiter Resonatorspiegel
- 16: OPO-Kristall
- 18: Frequenzmischereinrichtung
- 20: Mischer-Kristall
- 22: OPO-Strahlung
- 24: Einkoppeleinrichtung
- 26: Auskoppeleinrichtung
- 28: Einkoppelspiegel
- 29: Breitband-Einkoppelspiegel
- 30: Umlenkspiegel
- 32: Auskoppelspiegel
- 34: abstimmbare Strahlquelle
- 36: zweiter optisch parametrischer Oszillator
- 38: optischer Resonator
- 40: dritter Resonatorspiegel
- 41: vierter Resonatorspiegel
- 42: OPO-Kristall
- 44: Frequenzmischereinrichtung
- 46: Mischerkristall
- 48: Einkoppeleinrichtung
- 50: Einkoppelspiegel
- 52: Frequenzkonverter
- 54: Strahlteiler
- 56: Umlenkeinrichtung
- 58: optische Verzögerungsstrecke
- 60: Laseranordnung
- λₚᵤₘₚ: Pumpstrahlung
- λₘᵢₓ: Mischerstrahlung
- λₘᵢₓ₂: Mischstrahlung
- λᵣₑₛᵤₗₜ: resultierende Strahlung
- λ_{signal}: Signalstrahlung
- λᵤᵥ: UV-Strahlung

## Patentansprüche

1. Nichtlinear-optischer Frequenzkonverter (52) mit einem ersten optisch parametrischen Oszillator (10), OPO,
der einen optischen Resonator (12) mit Resonatorspiegeln (14, 15) und einen nichtlinearen optischen OPO-Kristall (16) zur Erzeugung einer OPO-Strahlung (22), wobei der optische Resonator (12) dazu ausgebildet ist, die OPO-Strahlung zwischen den Resonatorspiegeln (14, 15) hin und her schwingen zu lassen, und eine im Inneren des Resonators (12) zwischen den zwei Resonatorspiegeln (14, 15) vorgesehene Frequenzmischereinrichtung (18) mit einem nichtlinearen optischen Mischer-Kristall (20) zum Mischen der OPO-Strahlung (22) mit einer Mischerstrahlung (λₘᵢₓ), um durch Summenfrequenzerzeugung eine resultierende Strahlung (λᵣₑₛᵤₗₜ) zu erzeugen, aufweist,
wobei der erste optisch parametrische Oszillator (10) eine Einkoppeleinrichtung (24) aufweist und wobei eine abstimmbare Strahlquelle (34) zur Lieferung abstimmbarer Mischerstrahlung (λₘᵢₓ) vorgesehen ist, die über die Einkoppeleinrichtung (24) als Mischerstrahlung in die Frequenzmischereinrichtung (18) einkoppelbar ist, **dadurch gekennzeichnet,**
**dass** die abstimmbare Strahlquelle (34) einen zweiten optisch parametrischen Oszillator (36) aufweist.

2. Frequenzkonverter nach Anspruch 1,
**dadurch gekennzeichnet,**
**dass** er ein gepulster Frequenzkonverter (52) ist.

3. Frequenzkonverter nach einem der voranstehenden Ansprüche,
**dadurch gekennzeichnet,**
**dass** der erste optisch parametrische Oszillator (10) und die abstimmbare Strahlquelle (34) synchron gekoppelt sind.

4. Frequenzkonverter nach Anspruch 3,
**dadurch gekennzeichnet,**
**dass** eine gemeinsame Pulsquelle vorgesehen ist, um eine gemeinsame Pumpstrahlung (λₚᵤₘₚ) sowohl zu dem ersten als auch dem zweiten optisch parametrischen Oszillator zu liefern.

5. Frequenzkonverter nach einem der voranstehenden Ansprüche,
**dadurch gekennzeichnet,**
**dass** der zweite optisch parametrische Oszillator (36) ein abstimmbarer optisch parametrischer Oszillator mit resonatorinterner Summenfrequenzerzeugung ist.

6. Frequenzkonverter nach einem der voranstehenden Ansprüche,
**dadurch gekennzeichnet,**
**dass** die Einkoppeleinrichtung (24) einen Einkoppelspiegel (29) zum Einkoppeln der abstimmbaren Mischerstrahlung (λₘᵢₓ) in den die Frequenzmischereinrichtung (18) enthaltenden Resonator (12) des ersten optisch parametrischen Oszillators (10) aufweist
und **dass** der Einkoppelspiegel (29) derart breitbandig ausgebildet ist, dass er zumindest für einen Großteil des Frequenzbereichs oder für den gesamten Frequenzbereich der abstimmbaren Strahlungsquelle (34) entweder durchlässig oder reflektierend ist.

7. Frequenzkonverter nach Anspruch 6,
**dadurch gekennzeichnet,**
**dass** der Einkoppelspiegel (29) einen Reflexionsgrad von mehr als 90 %, vorzugsweise von mehr als 95 %, für den Frequenzbereich der OPO-Strahlung (22) des ersten optisch parametrischen Oszillators (10) und einen Transmissionsgrad von mehr als 50%, vorzugsweise von mehr als 90 %, für den Frequenzbereich der einzukoppelnden abstimmbaren Mischerstrahlung (λₘᵢₓ) aufweist.

8. Frequenzkonverter nach Anspruch 6,
**dadurch gekennzeichnet,**
**dass** der Einkoppelspiegel einen Transmissionsgrad von mehr als 90 %, vorzugsweise von mehr als 95 %, für den Frequenzbereich der OPO-Strahlung (22) des ersten optisch parametrischen Oszillators (10) und einen Reflexionsgrad von mehr als 50%, vorzugsweise von mehr als 90 %, für den Frequenzbereich der einzukoppelnden abstimmbaren Mischerstrahlung (λₘᵢₓ) aufweist.

9. Frequenzkonverter nach einem der Ansprüche 6 bis 8,
**dadurch gekennzeichnet,**
**dass** der Einkoppelspiegel (29) unter einem Winkel von etwa 45° zur Strahlung (22) des ersten optisch parametrischen Oszillators (10) steht.

10. Laseranordnung (60) zur Erzeugung gepulster abstimmbarer Laserstrahlung, **gekennzeichnet durch** einen Frequenzkonverter (52) nach einem der voranstehenden Ansprüche.

11. lonenmobilitätsspektrometrieverfahren, **gekennzeichnet durch** Verwendung einer Laseranordnung (60) nach Anspruch 10 zur Ionisierung von Molekülen.

12. LIDAR-Anordnung, **gekennzeichnet durch** einen Frequenzkonverter nach einem der Ansprüche 1 bis 9.

13. Verfahren zur Erzeugung gepulster abstimmbarer Laserstrahlung (λᵣₑₛᵤₗₜ; λᵤᵥ) unter Anwendung eines optisch nichtlinearen Prozesses in einem ersten optisch parametrischen Oszillator (10), der einen optischen Resonator (12) mit Resonatorspiegeln (14, 15) und einen nichtlinearen optischen OPO-Kristall (16) zur Erzeugung einer OPO-Strahlung (22), wobei der optische Resonator (12) dazu ausgebildet ist, die OPO-Strahlung zwischen den Resonatorspiegeln (14, 15) hin und her schwingen zu lassen, und eine im Inneren des Resonators (12) zwischen den zwei Resonatorspiegeln (14, 15) vorgesehene Frequenzmischereinrichtung (18) mit einem nichtlinearen optischen Mischer-Kristall (20) zum Mischen der OPO-Strahlung (22) mit einer Mischerstrahlung (λₘᵢₓ), um durch Summenfrequenzerzeugung eine resultierende Strahlung (λᵣₑₛᵤₗₜ) zu erzeugen, aufweist,
mit dem Schritt: Einkoppeln einer in der Wellenlänge abstimmbaren Strahlung (λₘᵢₓ) als Mischerstrahlung in die Frequenzmischeinrichtung (18) des ersten otisch parametrischen Oszillators (10),
**gekennzeichnet durch**
Erzeugung der in der Wellenlänge abstimmbaren einzukoppelnden Mischerstrahlung (λₘᵢₓ) mittels eines zweiten optisch parametrischen Oszillators (36).

## Claims

1. Non-linear optical frequency converter (52), having a first optically parametric oscillator (10), OPO, which has an optical resonator (12) with resonator mirrors (14, 15) and a non-linear optical OPO crystal (16) for generating OPO radiation (22), wherein the optical resonator (12) is designed to allow the OPO radiation to oscillate back and forth between the resonator mirrors (14, 15), and a frequency mixer device (18) provided in the interior of the resonator (12) between the two resonator mirrors (14, 15), said frequency mixer device having a non-linear optical mixer crystal (20) for mixing the OPO radiation (22) with mixer radiation (λₘᵢₓ) in order to generate, through sum frequency generation, a resulting radiation (λᵣₑₛᵤₗₜ), wherein the first optically parametric oscillator (10) has a coupling-in device (24), and wherein a tunable radiation source (34) is provided for supplying tunable mixer radiation (λₘᵢₓ) which can be coupled into the frequency mixer device (18) as mixer radiation via the coupling-in device (24), **characterized in that** the tunable radiation source (34) has a second optically parametric oscillator (36).

2. Frequency converter according to claim 1, **characterized in that** it is a pulsed frequency converter (52).

3. Frequency converter according to any one of the preceding claims, **characterized in that** the first optically parametric oscillator (10) and the tunable radiation source (34) are synchronously coupled.

4. Frequency converter according to claim 3, **characterized in that** a common pulse source is provided for supplying a common pump radiation (λₚᵤₘₚ) both to the first and to the second optically parametric oscillator.

5. Frequency converter according to any one of the preceding claims, **characterized in that** the second optically parametric oscillator (36) is a tunable optically parametric oscillator with resonator-internal sum frequency generation.

6. Frequency converter according to any one of the preceding claims, **characterized in that** the coupling-in device (24) has a coupling-in mirror (29) for coupling the tunable mixer radiation (λₘᵢₓ) into the resonator (12), containing the frequency mixer device (18), of the first optically parametric oscillator (10), and **in that** the coupling-in mirror (29) is of broadband type so that it is either transparent or reflective at least to a large part of the frequency range or to the entire frequency range of the tunable radiation source (34).

7. Frequency converter according to claim 6, **characterized in that** the coupling-in mirror (29) has a degree of reflection of more than 90%, preferably of more than 95%, for the frequency range of the OPO radiation (22) of the first optically parametric oscillator (10) and a degree of transmission of more than 50%, preferably of more than 90%, for the frequency range of the tunable mixer radiation (λₘᵢₓ) that is to be coupled in.

8. Frequency converter according to claim 6, **characterized in that** the coupling-in mirror has a degree of transmission of more than 90%, preferably of more than 95%, for the frequency range of the OPO radiation (22) of the first optically parametric oscillator (10) and a degree of reflection of more than 50%, preferably of more than 90%, for the frequency range of the tunable mixer radiation (λₘᵢₓ) that is to be coupled in.

9. Frequency converter according to any one of claims 6 to 8, **characterized in that** the coupling-in mirror (29) is at an angle of approximately 45° to the radiation (22) of the first optically parametric oscillator (10).

10. Laser arrangement (60) for generating pulsed tunable laser radiation, **characterized by** a frequency converter (52) according to any one of the preceding claims.

11. Ion mobility spectrometry method, **characterized by** the use of a laser arrangement (60) according to claim 10 for ionizing molecules.

12. LIDAR arrangement, **characterized by** a frequency converter according to any one of claims 1 to 9.

13. Method for generating pulsed tunable laser radiation (λᵣₑₛᵤₗₜ; λᵤᵥ) using an optically non-linear process in a first optically parametric oscillator (10) which has an optical resonator (12) with resonator mirrors (14, 15) and a non-linear optical OPO crystal (16) for generating OPO radiation (22), wherein the optical resonator (12) is designed to allow the OPO radiation to oscillate back and forth between the resonator mirrors (14, 15), and a frequency mixer device (18) provided in the interior of the resonator (12) between the two resonator mirrors (14, 15), said frequency mixer device having a non-linear optical mixer crystal (20) for mixing the OPO radiation (22) with mixer radiation (λₘᵢₓ) in order to generate, through sum frequency generation, a resulting radiation (λᵣₑₛᵤₗₜ), said method comprising the step: coupling a wavelength-tunable radiation (λₘᵢₓ) as mixer radiation into the frequency mixer device (18) of the first optically parametric oscillator (10), **characterized by** generating by means of a second optically parametric oscillator (36) the wavelength-tunable mixer radiation (λₘᵢₓ) that is to be coupled in.

## Revendications

1. Convertisseur de fréquence optique non linéaire (52) avec un premier oscillateur à paramètres optiques (10), OPO, qui comprend un résonateur optique (12) doté de miroirs de résonateur (14, 15) et un cristal optique non linéaire OPO (16) pour la génération d'un rayonnement OPO (22), dans lequel le résonateur optique (12) est conçu pour laisser osciller le rayonnement OPO d'un miroir de résonateur à l'autre (14, 15), et un dispositif mélangeur de fréquences (18) prévu à l'intérieur du résonateur (12) entre les deux miroirs de résonateur (14, 15) et doté d'un cristal mélangeur optique non linéaire (20) pour le mélange du rayonnement OPO (22) avec un rayonnement du mélangeur (λₘᵢₓ) afin de générer un rayonnement résultant (λᵣₑₛᵤₗₜ) par la génération d'une fréquence sommée,
dans lequel le premier oscillateur à paramètres optiques (10) comprend un dispositif de couplage (24) et il est prévu une source de rayonnement accordable (34) pour délivrer un rayonnement de mélangeur accordable (λₘᵢₓ) par le biais duquel le dispositif de couplage (24) peut être couplé en tant que rayonnement du mélangeur dans le dispositif mélangeur de fréquences (18), **caractérisé en ce que**,
la source de rayonnement accordable (34) comprend un second oscillateur à paramètres optiques (36).

2. Convertisseur de fréquence selon la revendication 1,
**caractérisé en ce qu'**il est un convertisseur de fréquence pulsé (52).

3. Convertisseur de fréquence selon l'une quelconque des revendications précédentes,
**caractérisé en ce que** le premier oscillateur à paramètres optiques (10) et la source de rayonnement accordable (34) sont couplés de manière synchrone.

4. Convertisseur de fréquence selon la revendication 3,
**caractérisé en ce qu'**il est prévu une source d'impulsions commune pour fournir un rayonnement de pompage commun (λₚᵤₘₚ) aussi bien au premier qu'au second oscillateur à paramètres optiques.

5. Convertisseur de fréquence selon l'une quelconque des revendications précédentes,
**caractérisé en ce que** le second oscillateur à paramètres optiques (36) est un oscillateur à paramètres optiques accordable avec une génération d'une fréquence sommée interne au résonateur.

6. Convertisseur de fréquence selon l'une quelconque des revendications précédentes,
**caractérisé en ce que** le dispositif de couplage (24) comprend un miroir de couplage (29) pour le couplage du rayonnement de mélangeur accordable (λₘᵢₓ) dans le résonateur (12) du premier oscillateur à paramètres optiques (10) contenant le dispositif mélangeur de fréquences (18)
et que le miroir de couplage (29) est formé avec une largeur de bande de manière à être soit transparent, soit réfléchissant au moins pour une grande partie de la plage de fréquences ou pour toute la plage de fréquences de la source de rayonnement accordable (34).

7. Convertisseur de fréquence selon la revendication 6,
**caractérisé en ce que** le miroir de couplage (29) présente un degré de réflexion de plus de 90 %, de préférence de plus de 95 %, pour la plage de fréquences du rayonnement OPO (22) du premier oscillateur à paramètres optiques (10) et un degré de transmission de plus de 50 %, de préférence de plus de 90 %, pour la plage de fréquences du rayonnement du mélangeur accordable (λₘᵢₓ) à coupler.

8. Convertisseur de fréquence selon la revendication 6,
**caractérisé en ce que** le miroir de couplage présente un degré de transmission de plus de 90 %, de préférence de plus de 95 %, pour la plage de fréquences du rayonnement OPO (22) du premier oscillateur à paramètres optiques (10) et un degré de réflexion de plus de 50 %, de préférence de plus de 90 %, pour la plage de fréquences du rayonnement du mélangeur accordable (λₘᵢₓ) à coupler.

9. Convertisseur de fréquence selon l'une quelconque des revendications 6 à 8,
**caractérisé en ce que** le miroir de couplage (29) forme un angle d'environ 45° avec le rayonnement (22) du premier oscillateur à paramètres optiques (10).

10. Dispositif laser (60) pour la génération d'un rayonnement laser accordable pulsé, **caractérisé par** un convertisseur de fréquence (52) selon l'une quelconque des revendications précédentes.

11. Procédé de spectrométrie de mobilité ionique, **caractérisé par** l'emploi d'un dispositif laser (60) selon la revendication 10 pour l'ionisation de molécules.

12. Dispositif LIDAR, **caractérisé par** un convertisseur de fréquence selon l'une quelconque des revendications 1 à 9.

13. Procédé de génération d'un rayonnement laser accordable pulsé (λᵣₑₛᵤₗₜ ; λᵤᵥ) à l'aide d'un processus optiquement non linéaire dans un premier oscillateur à paramètres optiques (10), qui comprend un résonateur optique (12) doté de miroirs de résonateur (14, 15) et un cristal optique non linéaire OPO (16) pour la génération d'un rayonnement OPO (22), dans lequel le résonateur optique (12) est conçu pour laisser osciller le rayonnement OPO d'un miroir de résonateur à l'autre (14, 15), et un dispositif mélangeur de fréquences (18) prévu à l'intérieur du résonateur (12) entre les deux miroirs de résonateur (14, 15) et doté d'un cristal mélangeur optique non linéaire (20) pour le mélange du rayonnement OPO (22) avec un rayonnement du mélangeur (λₘᵢₓ) afin de générer un rayonnement résultant (λᵣₑₛᵤₗₜ) par la génération d'une fréquence sommée, avec l'étape de : couplage d'un rayonnement (λₘᵢₓ), accordable dans la longueur d'onde, en tant que rayonnement du mélangeur dans le dispositif mélangeur de fréquences (18) du premier oscillateur à paramètres optiques (10),
**caractérisé par** la génération du rayonnement du mélangeur (λₘᵢₓ) à coupler accordable dans la longueur d'onde au moyen d'un second oscillateur à paramètres optiques (36).
